# EUROPÄISCHE PATENTSCHRIFT

(11) **EP 3 572 803 B1**
(45) Veröffentlichungstag und Bekanntmachung des Hinweises auf die Patenterteilung: **08.01.2025**
(21) Anmeldenummer: 19172499.6
(22) Anmeldetag: 03.05.2019
(51) Int. Cl.: G01N 21/89, D06H 3/08, G01N 33/36, D01H 13/16, G01B 11/10

(54) **GARNSENSOR ZUM OPTISCHEN ERFASSEN EINES IN SEINER LÄNGSRICHTUNG BEWEGTEN GARNS**
YARN SENSOR FOR OPTICAL DETECTION OF A YARN MOVING IN A LONGITUDINAL DIRECTION
CAPTEUR DE FIL DESTINÉ À LA DÉTECTION OPTIQUE D'UN FIL SE DÉPLAÇANT DANS SA DIRECTION LONGITUDINALE

(30) Priorität: 15.05.2018 DE 102018111648
(43) Veröffentlichungstag der Anmeldung: 27.11.2019
(73) Patentinhaber: Saurer Intelligent Technology AG, 9320 Arbon Thurgau (CH)
(72) Erfinder: Werheit, Patrick, 52066 Aachen (DE)
(74) Vertreter: Schniedermeyer, Markus

(56) Entgegenhaltungen:
- WO-A1-2016/027792
- DE-A1- 4 122 305
- US-A- 4 490 618

## Beschreibung

Der Gegenstand der Erfindung bezieht sich auf einen Garnsensor zum optischen Erfassen eines in seiner Längsrichtung bewegten Garns. Die Erfindung betrifft ebenfalls eine Textilmaschine mit dem Garnsensor und ein Verfahren zum Betreiben eines Garnsensors.

Garnsensoren werden an Textilmaschinen, wie Spinnmaschinen und Spulmaschinen, eingesetzt. Sie dienen zur Überwachung des Garns und/oder der Garnqualität. Derartige Garnsensoren weisen eine Lichtquelle und einen Detektor auf, wodurch ein optisch arbeitendes Überwachungssystem bereitgestellt wird, um eine entsprechende Signalerfassung zu gewährleisten. Die berührungslose Überwachung und Abtastung des Garns erfolgt für jede Arbeitsstelle getrennt. Die Verwendung eines Garnüberwachungssystems ist für die Textilmaschine und somit auch für die Qualität des Garns von besonderer Bedeutung.

Das Garnüberwachungssystem liefert eine Information über ein Vorhandensein des Garns. In einem solchen Fall bildet der Garnsensor einen sogenannten Fadenwächter. Es sind auch Garnsensoren bekannt, mittels denen die Garnstärke bzw. Garnqualität überwacht werden.

Durch das optische Erfassen des Garnes kann also das Vorhandensein des Garnes überwacht oder es kann die Qualität des Garnes ermittelt werden. Zur Ermittlung der Qualität des Garnes werden insbesondere der Durchmesser des Garnes bestimmt und Verunreinigungen, insbesondere Fremdfasern, detektiert.

Die US 4,490,618 offenbart eine Vorrichtung zur Analyse einer Oberfläche eines textilen Flächengewebes mit einem Prisma mit einer ersten und einer zweiten brechenden Oberfläche und einer Rückfläche. Eine brechende Oberfläche ist ausgebildet, um unter vorbestimmten Druck mit dem textilen Flächengewebe in Kontakt zu kommen.

Gemäß der WO 2016/027792A1 wird ein gewebter oder gestrickter Stoff mit einer Totalreflexionsmessmethode gemessen.

Bei den bekannten optischen Garnsensoren wird das Garn innerhalb eines Messspalts berührungslos geführt. Durch das Garn und die mit dem Garn eingeschleppte Luft werden Staub, Schmutzpartikel und Avivagen zu dem Garnsensor geführt. Diese Partikel lagern sich auf den Sensorflächen des Garnsensors ab, wodurch es zu einem Verschmutzungsgrad kommen kann, der zu erheblichen Verfälschungen von Messwerten führt.

Die DE 41 22 305 A1 offenbart eine Vorrichtung zur optoelektrischen Abtastung eines Fadens mit einem Fadenführungselement, um Flatterbewegungen des Fadens zu vermeiden. Die optoelektrische Abtastung gebraucht einen Reflexions-Sensor, wobei ein Sensor vorgesehen ist, der als Licht emittierendes Bauelement eine Fotodiode mit vorgesetzter Sammellinse und als Licht empfangendes Bauelement einen zentral in der Sammellinse angeordneten Lichtleiter mit zugeordnetem Fotodetektor aufweist.

Auch ist das Verschmutzungsproblem bei Garnsensoren bereits erkannt worden. Durch die EP 1 655 599 B1 ist ein Garnsensor zum optischen Abtasten eines in seiner Längsrichtung in einem Messspalt bewegten Garns bekannt, bei dem im Wesentlichen lediglich vom Garn reflektiertes Licht einer Lichtquelle zwei Empfänger für reflektiertes Licht erreicht. Hierdurch lassen sich Fremdeinflüsse, bspw. durch Fasern oder Staub, verringern.

Durch die Druckschrift WO 93/12028 A1 ist bspw. bekannt, dass die Sensorflächen des Garnsensors gereinigt werden. Um eine Reinigung der Sensorflächen zu erreichen, wird das Garn bis auf einen geringen fest eingestellten Abstand zu der Sensorfläche hin bewegt. Da das Garn eine gewisse Haarigkeit aufweist, wird ein "Pinseleffekt" gegenüber der optischen Innenfläche erreicht. Um das laufende Garn entsteht durch eine Grenzschichtströmung ein Luftzug, der gleichfalls einen Reinigungseffekt haben kann.

Hiervon ausgehend liegt der vorliegenden Erfindung die Zielsetzung zugrunde, die Zuverlässigkeit des Garnsensors zu erhöhen.

Diese Aufgabe wird erfindungsgemäß durch einen Garnsensor mit den Merkmalen des Anspruchs 1 gelöst. Vorteilhafte Weiterbildungen und Ausgestaltungen des erfindungsgemäßen Garnsensors sind Gegenstand der abhängigen Ansprüche.

Die in den abhängig formulierten Ansprüchen einzeln aufgeführten Merkmale können in beliebiger, technologisch sinnvoller Weise miteinander kombiniert werden und weitere Ausgestaltungen der Erfindung definieren. Darüber hinaus werden die in den Ansprüchen angegebenen Merkmale in der Beschreibung näher präzisiert und erläutert, wobei weitere bevorzugte Ausführungsbeispiele der Erfindung dargestellt werden.

Erfindungsgemäß wird ein Garnsensor zum optischen Erfassen eines in seiner Längsrichtung bewegten Garns mit einer Lichtquelle, einem Detektor und einem Lichtführungsmittel zur Führung des von der Lichtquelle ausgesandten Lichtes vorgeschlagen. Es sind Garnführungsmittel vorhanden, um ein Garn mit der Außenfläche des Lichtführungsmittels in Kontakt zu bringen. Die Lichtführungsmittel sind so ausgebildet, dass wenn kein Garn an der Außenfläche des Lichtführungsmittels anliegt, eine Totalreflektion erreicht wird und dass durch Kontakt mit dem Garn zumindest ein Teil des Lichtes aus dem Lichtführungsmittel ausgekoppelt wird.

Der Detektor ist so angeordnet und ausgebildet, dass er das Licht, welches aus dem Lichtführungsmittel ausgekoppelt wird, detektiert oder die verminderte Intensität im totalreflektierten Strahl bewertet.

Der erfindungsgemäße Garnsensor basiert dabei auf dem Effekt der frustrierten Totalreflektion (frustrated total internal reflection, FTIR). Eine Totalreflektion findet an einer Grenzfläche zweier Licht absorbierender Medien mit unterschiedlich großer Ausbreitungsgeschwindigkeit des Lichts statt, wenn der Einfallswinkel einen bestimmten Wert, den sogenannten Grenzwinkel der Totalreflektion, überschreitet. Die optische Dichte der Luft ist wesentlich geringer als die optische Dichte des Garns. Durch den Kontakt des Garns an einer Kontaktfläche zwischen Garn und Außenfläche des Lichtführungsmittels kommt es zur frustrierten Totalreflektion, da der Brechungsindex des Garns größer ist als der Brechungsindex der Luft.

Durch den Effekt der frustrierten Totalreflektion wird das Licht, welches im Kontaktbereich zwischen Garn und Außenfläche des Lichtführungsmittels aus dem Lichtführungsmittel austritt, am Garn gestreut. Das Streulicht propagiert aus dem Lichtführungsmittel heraus. Dieses Streulicht kann mittels des Detektors direkt oder indirekt detektiert werden, wodurch eine Erfassung des an der Außenfläche anliegenden Garns ermöglicht wird. Oder anders ausgedrückt es wird der Einfluss des Garnes auf das von der Lichtquelle ausgesendete Licht erfasst. Es gibt dazu prinzipiell zwei Möglichkeiten, die alternativ oder in Verbindung miteinander zur Anwendung kommen können.

Gemäß einer ersten Ausführungsform ist der Detektor so angeordnet und ausgebildet, dass er Licht erfasst, dass durch das Garn aus dem Lichtführungsmittel ausgekoppelt wird.

Gemäß einer zweiten Ausführungsform ist der Detektor so angeordnet und ausgebildet, dass er das verbleibende, durch das Lichtführungsmittel geführte und durch das Garn nicht ausgekoppelte Licht erfasst.

Bei der ersten erfindungsgemäßen Ausgestaltung des Garnsensors wird durch den Detektor das Streulicht, welches aus dem Lichtführungsmittel ausgekoppelt wird, detektiert. Das ist das Licht, welches an der Kontaktfläche zwischen Garn und Außenfläche des Lichtführungsmittels reflektiert wird. Alternativ oder zusätzlich besteht die Möglichkeit, dass das Lichtführungsmittel dazu geeignet und bestimmt ist, dass an der Außenfläche des Lichtführungsmittels reflektierte Licht zum Detektor zu führen. Mittels des Detektors wird dann die verminderte Intensität im totalreflektierten Strahl bewertet. Die Intensität im totalreflektierten Strahl ist im Wesentlichen um das aus dem Lichtführungsmittel ausgekoppelte gestreute Licht vermindert.

Durch die erfindungsgemäße Ausgestaltung des optischen Garnsensors wird die bisherige Entwicklung von optisch arbeitenden Garnsensoren verlassen und ein völlig neuer Weg eingeschlagen. Unter Ausnutzung des FTIR-Effekts wird ein Garnsensor bereitgestellt, welcher in seinem Aufbau einfach und gegenüber Umgebungseinflüssen unempfindlicher ist. Dadurch, dass im Betrieb das bewegte Garn an dem Lichtführungsmittel anliegt, werden die bei bekannten optischen Garnsensoren bekannte Probleme von Verschmutzungseinflüssen im Wesentlichen vermieden, da das Garn einen Selbstreinigungseffekt im Bereich der Kontaktfläche mit dem Lichtführungsmittel bewirkt.

Auch der der Kontaktfläche benachbarte Bereich des Lichtführungsmittels wird gleichfalls gereinigt. Durch die Haarigkeit des Garns werden Verschmutzungen nicht nur von der Kontaktfläche zwischen Garn und Außenfläche des Lichtführungsmittels beseitigt, sondern auch der der Kontaktfläche benachbarte Bereich. Dieser Reinigungseffekt wird durch einen "Kamineffekt" verstärkt, da das bewegte Garn und die sich bildendende Grenzschichtströmung einen Luftzug erzeugt.

Die erfindungsgemäße Ausgestaltung des Garnsensors ermöglicht auch eine sehr kompakte Bauform, da der Aufbau des optischen Systems auf eine lediglich sehr geringe Anzahl von Bauteilen minimiert werden kann.

Der erfindungsgemäße Garnsensor kann als ein sogenannter Fadenwächter verwendet werden. Der Fadenwächter liefert eine Information darüber, ob ein Garn vorhanden ist oder nicht.

Die Intensität des Streulichts, welches aus dem Lichtführungsmittel ausgekoppelt wird, ist proportional zur Kontaktfläche des Garns mit der Außenfläche des Lichtführungsmittels. Bei einer konstanten Fadenspannung kann auf den Durchmesser des Garns rückgeschlossen werden, so dass der Garnsensor auch eine Information über die Qualität des Garns zur Verfügung stellen kann. So können bspw. Knoten im Garn erfasst werden.

Unter der Voraussetzung, dass bspw. Fremdfasern, Staub etc. einen anderen Brechungsindex haben als der Garnkörper, so kann mittels des erfindungsgemäßen Garnsensors eine Detektion von Fremdfasern erreicht werden.

Als Garnführungsmittel kommt eine beliebige Anordnung in Frage. Entscheidend ist nur, dass das Garn mit dem Lichtführungsmittel in Kontakt gebracht wird. Dazu kann das Garnführungsmittel zum Beispiel in Fadenlaufrichtung vor und hinter dem Lichtführungsmittel jeweils eine Führungsöse beinhalten.

Im Hinblick darauf, dass das Garn an dem Lichtführungsmittel berührend geführt wird, ist es vorteilhaft, wenn das Lichtführungsmittel wenigstens teilweise aus einer transparenten Keramik besteht. Die Verwendung einer Keramik hat den Vorteil, dass der Verschleiß des Lichtführungsmittels gering gehalten werden kann. Die transparente Keramik kann eine Außenschicht eines Lichtführungsmittels darstellen. So kann bspw. die transparente Keramik mit einem Glaskörper oder Kunststoffkörper verbunden sein, wobei die Verbindung bspw. mittels eines transparenten Klebers erfolgt. Wird eine transparente Keramik bspw. mit einem Kunststoffkörper verklebt, so ist sicherzustellen, dass in der Klebeschicht keine Lufteinschlüsse entstehen. Vorzugsweise ist die Außenfläche des Lichtführungsmittels konvex gebogen. Hierdurch wird eine zuverlässige Anlage des Garns an die Außenfläche des Lichtführungsmittels erreicht. Gemäß einer noch weiteren vorteilhaften Ausgestaltung hat das Lichtführungsmittel einen kreisförmigen Querschnitt. Das Lichtführungsmittel kann insbesondere in Form eines Rundstabs ausgebildet sein.

Die Lichtquelle ist vorzugsweise dazu geeignet und bestimmt, ein Infrarotlicht abzugeben. Insbesondere kann die Lichtquelle mindestens eine Leuchtdiode umfassen.

Der Detektor ist insbesondere ein räumlich auflösender Detektor. Dieser weist bspw. ein CCB oder CMOS Array, eine CCD oder CMOS Zeile auf. Insbesondere wird vorgeschlagen, dass der Detektor ein aktiver Pixelsensor ist. Der aktive Pixelsensor weist eine integrierte Schaltung mit einer Reihe von Pixelsensoren auf. Jeder Pixelsensor kann bspw. eine Fotodiode mit einem aktiven Verstärker umfassen.

Gemäß einer noch weiteren vorteilhaften Ausgestaltung des erfindungsgemäßen Garnsensors wird vorgeschlagen, dass das Lichtführungsmittel ein Bestandteil eines Garnführungselements ist. Ein so gebildeter Garnsensor hat den Vorteil, dass die Anzahl der Bauteile verringert werden kann. Darüber hinaus kann durch den kompakten Aufbau des Garnsensors, insbesondere auch dadurch, dass das Lichtführungsmittel ein Bestandteil eines Garnführungselements ist, eine höhere Überwachungsqualität im Spinnprozess erreicht werden.

Die Erfindung betrifft außerdem eine Textilmaschine mit einem erfindungsgemäßen Garnsensor.

Weiter wird ein Verfahren zum Betreiben eines erfindungsgemäßen Garnsensors vorgeschlagen. Die Erfindung betrifft damit ebenfalls ein Verfahren zum optischen Erfassen eines in seiner Längsrichtung bewegten Garnes mit einem Lichtführungsmittel. Gemäß dem erfindungsgemäßen Verfahren wird Licht durch das Lichtführungsmittel geführt, wobei wenn kein Garn an der Außenfläche des Lichtführungsmittels anliegt, eine Totalreflektion erreicht wird. Das Garn wird mit der Außenfläche des Lichtführungsmittels in Kontakt gebracht, so dass zumindest ein Teil des Lichtes aus dem Lichtführungsmittel ausgekoppelt wird. Schließlich wird das vom Garn beeinflusste Licht erfasst. Dazu kann das Licht erfasst werden, dass durch das Garn aus dem Lichtführungsmittel ausgekoppelt wird. Es ist auch möglich, das verbleibende, durch das Lichtführungsmittel geführte und durch das Garn nicht ausgekoppelte Licht zu erfassen.

Das so erfasste vom Garn beeinflusste Licht kann ausgewertet werden. So lassen sich Durchmesserabweichungen und Verunreinigungen erfassen. Es kann auch nur das Vorhandensein des Garns bzw. das Fadens ausgewertet werden.

Weitere Vorteile und Einzelheiten des erfindungsgemäßen Garnsensors werden anhand zweier bevorzugter Ausführungsbeispiele erläutert, ohne dass der Gegenstand der Erfindung auf diese konkreten Ausführungsbeispiele beschränkt wird. Es zeigen:
- Fig. 1:: eine Prinzipdarstellung einer Arbeitsstelle,
- Fig. 2:: schematisch ein erstes Ausführungsbeispiel eines Garnsensors,
- Fig. 3:: schematisch ein zweites Ausführungsbeispiel eines Garnsensors, und
- Fig. 4:: eine Momentaufnahme des Garns und ein zur Momentaufnahme dazugehöriges Diagramm einer Auswertung eines Signals eines Garnsensors.

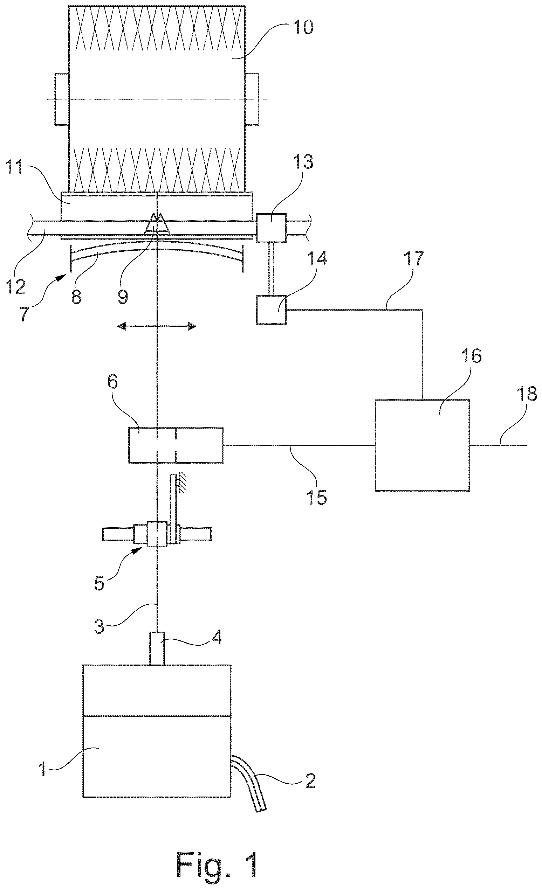

Fig. 1 zeigt eine Prinzipdarstellung einer Arbeitsstelle einer erfindungsgemäßen Textilmaschine. Die Textilmaschine ist in dem Ausführungsbeispiel als Offenend-Spinnmaschine ausgebildet. Einer Spinnbox 1 wird ein Faserband 2 zugeführt. Das in der Spinnbox 2 erzeugte Garn 3 wird über ein Abzugsröhrchen 4 mittels eines Abzugswalzenpaars 5 abgezogen. Das Garn 3 durchläuft einen erfindungsgemäßen Garnsensor 6 und wird über einen Bügel 8 durch eine Hin- und Herbewegung eines Fadenführers 9 einer Changiereinrichtung 7 über eine vorgegebene Breite zu einer Kreuzspule 10 aufgewickelt.

Eine Friktionswalze 11 treibt die Kreuzspule 10 an. Der Fadenführer 9 ist auf einer Fadenführungsstange 12 angeordnet. Die Fadenführungsstange 12 wird von einem Fadenführungsgetriebe 13 hin und her bewegt. Zur Erzeugung der Hin- und Herbewegung des Fadenführergetriebes 13 ist eine Antriebseinheit 14 vorgesehen.

Aus der Darstellung nach Fig. 1 ist ersichtlich, dass der Garnsensor 6 zur Überwachung des laufenden Garns 3 oberhalb des Abzugswalzenpaars 5 im Changierbereich des Garns 3 angeordnet ist. Dies ist nicht zwingend. Es besteht auch die Möglichkeit, dass der Garnsensor 6 dem Abzugswalzenpaar 5 vorgeordnet ist. Die Anordnung des Garnsensors 6 ist hier beispielhaft dargestellt. Der Garnsensor 6 kann an geeigneten Stellen des Garnlaufs vorgesehen sein.

Der Garnsensor 6 ist über eine Signalleitung 15 mit einer Steuereinrichtung 16 verbunden. Die Steuereinrichtung 16 ist über eine weitere Signalleitung 17 mit der Antriebseinheit 14 verbunden. Bei der Antriebseinrichtung 14 handelt es sich vorzugsweise um einen Elektromotor. Über eine Signalleitung 18 kann die Steuereinrichtung 16 mit weiteren nicht dargestellten Spinnstellen, Datenverarbeitungseinrichtungen und Spinnmaschinen verbunden sein.

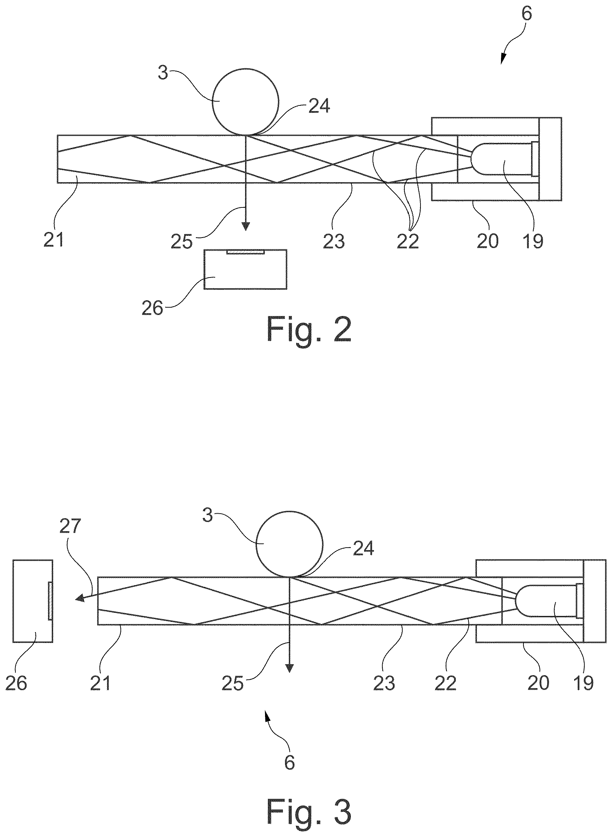

In der Fig. 2 ist ein erstes Ausführungsbeispiel eines Garnsensors 6 dargestellt. Der Garnsensor 6 weist eine Lichtquelle 19 auf. Bei der Lichtquelle 19 handelt es sich vorzugsweise um eine Infrarot-LED. Die Lichtquelle 19 ist in einem Gehäuse 20 angeordnet. In dem dargestellten Ausführungsbeispiel dient das Gehäuse 19 gleichzeitig als Halterung für ein Lichtführungsmittel 21. Das Lichtführungsmittel 21 ist in Form eines Rundstabs ausgebildet. Das Material des Lichtführungsmittels 21 ist vorzugsweise eine transparente Keramik.

Mit dem Bezugszeichen 22 werden lediglich zur Verdeutlichung des Prinzips der Erfindung Strahlen bezeichnet, die die Lichtquelle 19 emittiert. Die Strahlen 22 werden an der Außenfläche 23 reflektiert. Die Lichtquelle 19 und das Lichtführungsmittel 21 sind so ausgebildet, dass wenn kein Garn an der Außenfläche 23 des Lichtführungsmittels 21 anliegt, eine Totalreflektion erreicht wird.

In dem in der Fig. 2 dargestellten Ausführungsbeispiel ist schematisch ein Garn 3 dargestellt. Das Garn 3 liegt an der Außenfläche 23 des Lichtführungsmittels 21 an. Zwischen dem Garn 3 und der Außenfläche 23 liegt eine Kontaktfläche 24. Durch den Kontakt des Garns 3 mit dem Lichtführungsmittel 21 kommt es im Bereich der Kontaktfläche 24 zur frustrierten Totalreflektion, da der Brechungsindex des Garns 3 größer ist als der Brechungsindex der umgebenden Luft des Garnsensors 6. Durch die frustrierte Totalreflektion wird das Licht, welches im Bereich der Kontaktfläche 24 aus dem Lichtführungsmittel 21 austritt, am Garn gestreut. Das Streulicht propagiert durch das Lichtführungsmittel 21, was durch den Strahl 25 angedeutet wird. Dieses Streulicht wird durch einen Detektor 26 detektiert. Der Detektor 26 liefert Signale, die der Steuereinrichtung 16 bspw. zugeführt werden können.

Fig. 3 zeigt ein zweites Ausführungsbeispiel eines Garnsensors. Der Garnsensor 6 umfasst eine Lichtquelle 19, die in einem Gehäuse 20 angeordnet ist. Das Gehäuse 20 dient gleichzeitig als Halterung für ein Lichtführungsmittel 21. Auch bei diesem Ausführungsbeispiel liegt das Garn 3 an einer Außenfläche 23 des Lichtführungsmittels 21 an. Der Unterschied zwischen der Ausführungsform nach Fig. 3 und der Ausführungsform nach Fig. 2 besteht in der Anordnung des Detektors 26 und der damit verbundenen Auswertungsmethodik. Anstelle des gestreuten Lichts, welches durch den Strahl 25 angedeutet werden soll, welches aus dem Lichtführungsmittel 21 ausgekoppelt wird, wird die verminderte Intensität im totalreflektierten Strahl 27 bewertet.

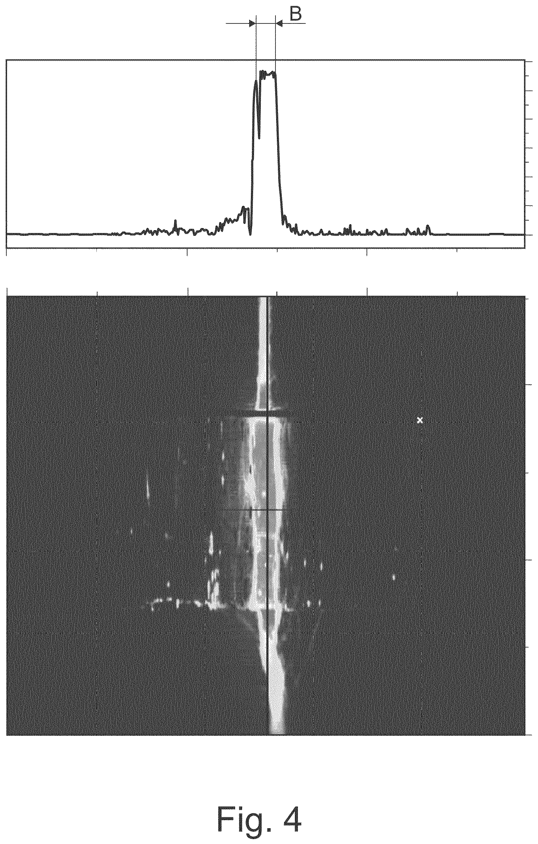

Der Detektor 26 ist vorzugsweise ein Pixelsensor, insbesondere ein aktiver Pixelsensor. In der Fig. 4 ist eine Momentaufnahme des Streulichts, welches ein Detektor erfasst hat, dargestellt. Die Auswertung dieser Aufnahme ist in einem Diagramm wiedergegeben. Es ist erkennbar, dass ein Garn an dem Lichtführungsmittel anliegt.

Aus der Breite B des Detektorsignals kann auf den Garndurchmesser geschlossen werden, da bei konstanter Fadenspannung das Streulicht proportional zum Garndurchmesser ist. Damit lassen sich auch Dünn- und Dickstellen im Garn detektieren.

Aus der zeitlichen Änderung des Detektorsignals kann auch eine Aussage darüber getroffen werden, ob sich die Spannung des Garns verändert hat.

### Bezugszeichenliste

- 1: Spinnbox
- 2: Faserband
- 3: Garn
- 4: Abzugsröhrchen
- 5: Abzugswalzenpaar
- 6: Garnsensor
- 7: Changiereinrichtung
- 8: Bügel
- 9: Fadenführer
- 10: Kreuzspule
- 11: Friktionswalze
- 12: Fadenführungsstange
- 13: Fadenführungsgetriebe
- 14: Antriebseinheit
- 15: Signalleitung
- 16: Steuereinrichtung
- 17: Signalleitung
- 18: Signalleitung
- 19: Lichtquelle
- 20: Gehäuse
- 21: Lichtführungsmittel
- 22: Strahlen
- 23: Außenfläche
- 24: Kontaktfläche
- 25: Strahl
- 26: Detektor
- 27: Strahl

## Patentansprüche

1. Garnsensor (6) zum optischen Erfassen eines in seiner Längsrichtung bewegten Garnes (3) mit einer Lichtquelle (19) und einem Detektor (26), umfassend ein Lichtführungsmittel (21) zur Führung des von der Lichtquelle (19) ausgesandten Lichtes und Garnführungsmittel um ein Garn (3) mit der Außenfläche (23) des Lichtführungsmittels (21) in Kontakt zu bringen,
wobei die Lichtführungsmittel (21) so ausgebildet sind, dass wenn kein Garn an der Außenfläche (23) des Lichtführungsmittels (21) anliegt, eine Totalreflektion erreicht wird und durch Kontakt mit dem Garn (3) zumindest ein Teil des Lichtes aus dem Lichtführungsmittel (21) ausgekoppelt wird und
der Detektor (26) so angeordnet und ausgebildet ist, dass er das Licht, welches aus dem Lichtführungsmittel (21) ausgekoppelt wird, detektiert oder die verminderte Intensität im totalreflektierten Strahl bewertet.

2. Garnsensor (6) nach Anspruch 1, **dadurch gekennzeichnet, dass** der Detektor (26) so angeordnet und ausgebildet ist, dass er Licht erfasst, dass durch das Garn (3) aus dem Lichtführungsmittel (21) ausgekoppelt wird.

3. Garnsensor (6) nach einem der Ansprüche 1 oder 2, **dadurch gekennzeichnet, dass** der Detektor (26) so angeordnet und ausgebildet ist, dass er das verbleibende, durch das Lichtführungsmittel (21) geführte und durch das Garn (3) nicht ausgekoppelte Licht erfasst.

4. Garnsensor (6) nach einem der vorherigen Ansprüche, **dadurch gekennzeichnet, dass** das Lichtführungsmittel (21) wenigstens teilweise aus einer transparenten Keramik besteht.

5. Garnsensor nach einem der vorherigen Ansprüche, **dadurch gekennzeichnet, dass** die Außenfläche des Lichtführungsmittels (21) konvex gebogen ist.

6. Garnsensor nach wenigstens einem der Ansprüche 1 bis 5, **dadurch gekennzeichnet, dass** der Detektor (26) ein räumlich auflösender Detektor ist.

7. Garnsensor nach wenigstens einem der Ansprüche 1 bis 5, **dadurch gekennzeichnet, dass** der Detektor (26) ein aktiver Pixelsensor ist.

8. Garnsensor nach einem der Ansprüche 1 bis 8, **dadurch gekennzeichnet, dass** das Lichtführungsmittel (21) ein Bestandteil eines Garnführungselementes ist.

9. Textilmaschine mit einem Garnsensor (6) nach einem der Ansprüche 1 bis 8.

10. Verfahren zum optischen Erfassen eines in seiner Längsrichtung bewegten Garnes (3) mit einem Lichtführungsmittel (21) umfassend die Schritte:
- führen von Licht durch das Lichtführungsmittel (21), wobei wenn kein Garn an der Außenfläche (23) des Lichtführungsmittels (21) anliegt, eine Totalreflektion erreicht wird,
- in Kontakt bringen des Garns (3) mit der Außenfläche (23) des Lichtführungsmittels (21), so dass zumindest ein Teil des Lichtes aus dem Lichtführungsmittel (21) ausgekoppelt wird und
- detektieren des Licht, welches aus dem Lichtführungsmittel (21) ausgekoppelt wird oder bewerten der verminderten Intensität im totalreflektierten Strahl.

## Claims

1. Yarn sensor (6) for optically sensing a yarn (3) moved in the longitudinal direction of the yarn (3), comprising a light source (19) and a detector (26), comprising a light guiding element (21) for guiding the light emitted by the light source (19) and yarn guiding elements in order to bring a yarn (3) into contact with the outer surface (23) of the light guiding element (21),
wherein the light guiding elements (21) are designed in such a way that if no yarn is in contact with the outer surface (23) of the light guiding element (21), total reflection is achieved and at least one part of the light is coupled out of the light guiding element (21) by contact with the yarn (3) and
the detector (26) is arranged and designed to detect the light that is coupled out of the light guiding element (21) or to evaluate the reduced intensity in the totally reflected beam.

2. Yarn sensor (6) according to claim 1, **characterised in that** the detector (26) is arranged and designed in such a way that the detector (26) senses light coupled out of the light guiding element (21) by the yarn (3).

3. Yarn sensor (6) according to claim 1 or 2, **characterised in that** the detector (26) is arranged and designed in such a way that the detector (26) senses the remaining light that is guided by the light guiding element (21) and is not coupled out by the yarn (3).

4. Yarn sensor (6) according to one of the preceding claims, **characterised in that** the light guiding element (21) consists at least partly of a transparent ceramic.

5. Yarn sensor according to one of the preceding claims, **characterised in that** the outer surface of the light guiding element (21) is convexly curved.

6. Yarn sensor according to at least one of claims 1 to 5, **characterised in that** the detector (26) is a spatially resolving detector.

7. Yarn sensor according to at least one of claims 1 to 5, **characterised in that** the detector (26) is an active pixel sensor.

8. Yarn sensor according to one of claims 1 to 8, **characterised in that** the light guiding element (21) is part of a yarn guiding element.

9. Textile machine, having a yarn sensor (6) according to one of claims 1 to 8.

10. Method for optically sensing a yarn (3) moved in the longitudinal direction of the yarn (3), by means of a light guiding element (21), comprising the following steps:
- guiding light by means of the light guiding element (21), wherein, if no yarn is in contact with the outer surface (23) of the light guiding element (21), total reflection is achieved
- bringing the yarn (3) into contact with the outer surface (23) of the light guiding element (21) so that at least one part of the light is coupled out of the light guiding element (21) and
- detecting the light that is coupled out from the light guiding element (21) or evaluating the reduced intensity in the totally reflected beam.

## Revendications

1. Capteur de fil (6) destiné à la détection optique d'un fil (3), déplacé dans sa direction longitudinale, à l'aide d'une source de lumière (19) et d'un détecteur (26), comprenant un moyen de guidage de lumière (21) destiné à guider la lumière émise par la source de lumière (19) et un moyen de guidage de fil pour amener un fil (3) en contact avec la surface extérieure (23) du moyen de guidage de lumière (21),
dans lequel le moyen de guidage de lumière (21) est conçu de telle sorte que, lorsqu'aucun fil n'est en contact avec la surface extérieure (23) du moyen de guidage de lumière (21), une réflexion totale est obtenue et, par contact avec le fil (3), au moins une partie de la lumière est extraite du moyen de guidage de lumière (21) et
le détecteur (26) est disposé et conçu de sorte qu'il détecte la lumière qui est extraite du moyen de guidage de lumière (21) ou qu'il évalue l'intensité atténuée dans le faisceau à réflexion totale.

2. Capteur de fil (6) selon la revendication 1, **caractérisé en ce que** le détecteur (26) est disposé et conçu de sorte qu'il détecte la lumière qui est extraite du moyen de guidage de lumière (21) par le fil (3).

3. Capteur de fil (6) selon l'une des revendications 1 ou 2, **caractérisé en ce que** le détecteur (26) est disposé et conçu de sorte qu'il détecte la lumière restante guidée par le moyen de guidage de lumière (21) et non extraite par le fil (3).

4. Capteur de fil (6) selon l'une des revendications précédentes, **caractérisé en ce que** le moyen de guidage de lumière (21) est constitué au moins partiellement d'une céramique transparente.

5. Capteur de fil selon l'une des revendications précédentes, **caractérisé en ce que** la surface extérieure du moyen de guidage de lumière (21) est courbée de manière convexe.

6. Capteur de fil selon au moins l'une des revendications 1 à 5, **caractérisé en ce que** le détecteur (26) est un détecteur à résolution spatiale.

7. Capteur de fil selon au moins l'une des revendications 1 à 5, **caractérisé en ce que** le détecteur (26) est un capteur à pixel actif.

8. Capteur de fil selon l'une des revendications 1 à 8, **caractérisé en ce que** le moyen de guidage de lumière (21) est un composant d'un élément de guidage de fil.

9. Machine textile comportant un capteur de fil (6) selon l'une des revendications 1 à 8.

10. Procédé permettant la détection optique d'un fil (3), déplacé dans sa direction longitudinale, à l'aide d'un moyen de guidage de lumière (21), comprenant les étapes consistant à :
- guider la lumière à travers le moyen de guidage de lumière (21), dans lequel, lorsqu'aucun fil n'est en contact avec la surface extérieure (23) du moyen de guidage de lumière (21), une réflexion totale est obtenue,
- mettre le fil (3) en contact avec la surface extérieure (23) du moyen de guidage de lumière (21), de sorte qu'au moins une partie de la lumière est extraite du moyen de guidage de lumière (21), et
- détecter la lumière qui est extraite du moyen de guidage de lumière (21) ou évaluer l'intensité atténuée dans le faisceau à réflexion totale.
